# EUROPEAN PATENT SPECIFICATION

(11) **EP 3 849 622 B1**
(45) Date of publication and mention of the grant of the patent: **11.05.2022**
(21) Application number: 19797246.6
(22) Date of filing: 29.10.2019
(51) Int. Cl.: A61L 9/12, B05B 11/00, B05B 12/14

(54) **A FRAGRANCE CARTRIDGE**
RIECHSTOFFKARTUSCHE
CARTOUCHE DE PARFUM

(30) Priority: 30.10.2018 EP 18382766
(43) Date of publication of application: 21.07.2021
(73) Proprietor: Noustique Perfumes, S.L., 08006 Barcelona (ES)
(72) Inventor: SOLER SAEZ, Pedro Antonio, 08784 PIERA (ES); LASALA ALONSO, Hugo, 50006 ZARAGOZA (ES); SUAREZ IRIBARNE, Alvaro, 08008 BARCELONA (ES); SOLER COSTA, Juan Ramon, 50003 ZARAGOZA (ES); CEAMANOS, Jesús, 50016 ZARAGOZA (ES)
(74) Representative: ZBM Patents - Zea, Barlocci & Markvardsen
(86) International application number: PCT/EP2019/079571
(87) International publication number: WO 2020/089253

(56) References cited:
- EP-A1- 2 233 214
- WO-A1-2006/004242
- WO-A1-2017/103634
- FR-A1- 2 857 339

## Description

The invention relates to cartridges, more specifically to liquid fragrance cartridges.

The present application claims the benefit and priority of EP 18 382 766.6, filed on October 30, 2018.

### BACKGROUND

Several cartridge concepts for dispensing devices are known. The cartridges usually comprise a fragrance container having a dispensing pump mechanism e.g., airless or 360°, so as to dispense the content.

Known solutions, however, involve several problems. Some cartridge arrangements, for instance, do not provide a good dosing accuracy or may have leakage problems which render the cartridge unreliable.

Sometimes, in order to provide e.g. a high dosing accuracy, the cartridge designs include complex fragrance containers and/or pump mechanisms involve high manufacturing costs, manufacturing and/or assembling difficulty and are not suitable for mass production.

There is a need to provide a fragrance cartridge having a simple design which provides a high dosing accuracy and reliability which at the same time is suitable for to mass production at reasonable costs.

WO2006/004242, on which the preamble of claim 1 is based, discloses a push-type dispensing device with an outer casing. A nozzle cap is fastened to a lower end of the outer casing. An inner casing is housed in the outer casing, contains contents, and moves vertically. A button is coupled to an upper end of the inner casing and actuated to move the inner casing downwards. A dispenser is coupled to a lower end of the inner casing and dispenses a prescribed amount of contents by a pumping action.

EP2233214 discloses a dispenser with a housing having curvilinear sides. A bore extends through the housing, which is adapted to receive a container. The container is adapted to dispense fluid through a first aperture in the top end of the housing. Fluid is dispensed from the container through the first aperture upon telescopically moving the housing by exerting a force against the at least one face of the curvilinear sides in a direction parallel to the longitudinal axis of the housing.

### SUMMARY

In a first aspect, a fragrance cartridge for a fragrance dispensing device is provided, as claimed in claim 1.

A fragrance cartridge according to the aspect enables the use of standard and/or commercially available fragrance containers e.g. made of glass, which additionally contain a pump mechanism to draw the content. Being able to use such containers in the cartridge enables adopting a simple and cheap solution. Moreover, the pump mechanism of such containers has a high dosing accuracy and prevents or at least reduces leakage problems. A reliable cartridge may thus be obtained.

In addition, the manufacturing process of the fragrance container and also of the housing may be implemented in mass production by known processes e.g. filling, assembling, etc. which substantially reduces the manufacturing costs of the cartridge.

Furthermore, the use of an internal adjusting element enables the implementation of an automatized manufacturing process e.g. as facilitates the insertion of the fragrance container into the housing. In addition, the adjusting element facilitates the retention of the fragrance container by snuggly fitting it inside the housing. The possibilities of rupture are thereby reduced while at the same time stability is provided. Furthermore, the use of an adjusting element provides the possibility of enclosing different fragrance containers i.e. having the different shapes, within the same housing.

Furthermore, due to the openings the cartridge enables preventing undesired actuating of the cartridge in which the released amount of liquid may not be registered. Thus, the remaining fragrance volume may reliably be measured.

In an example, the cartridge may comprise a read/write identification element attached to the housing. The cartridge may therefore be used in different external devices e.g. fragrance dispensing devices, and may also be used off line without having to access a certain data storage element e.g. arranged in an external device, to retrieve information, e.g. to determine the remaining fragrance amount in each cartridge.

In an example, the cartridge may comprise an authentication element arranged on the housing.

In a further aspect a fragrance dispensing device as claimed in claim 8 is provided.

### BRIEF DESCRIPTION OF THE DRAWINGS

Non-limiting examples of the present disclosure will be described in the following, with reference to the appended drawings, in which:
Figure 1 schematically illustrates a simplified longitudinal view of a cartridge according to an example;
Figure 2 schematically illustrates a fragrance container according to an example;
Figure 3 schematically illustrates a cartridge housing according to an example; and
Figure 4 illustrates a very schematic and simplified longitudinal view of a dispensing device according to an example.

### DETAILED DESCRIPTION

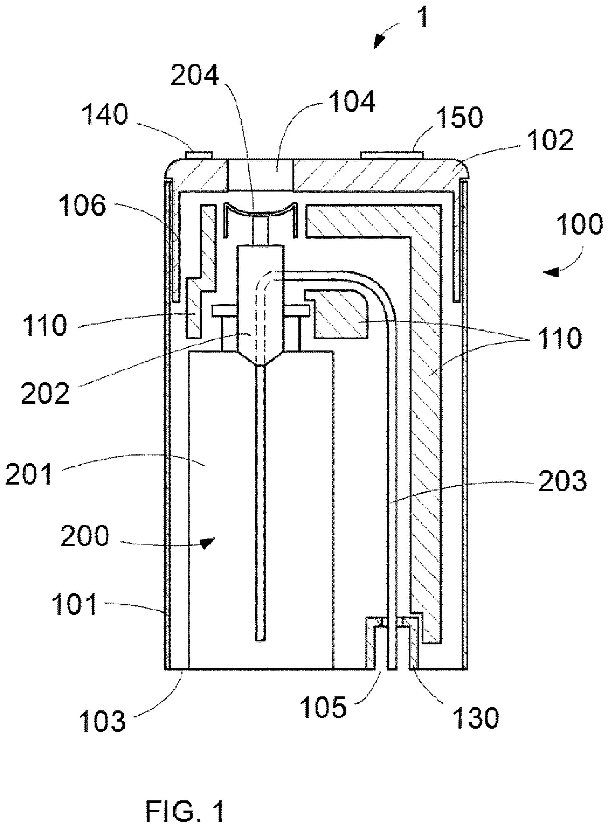

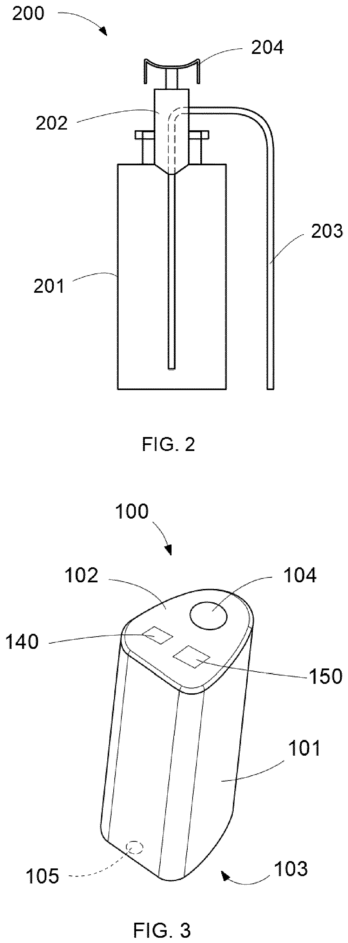

Figure 1 shows a longitudinal section of a cartridge 1 which comprises a housing 100 and a liquid fragrance container 200 inserted therein. For the sake of clarity, both elements would be firstly separately explained in Figures 2 and 3.

Figure 2 depicts a liquid fragrance container 200 which comprises a receptacle 201 for containing a liquid fragrance. The receptacle 201 may be elongated and substantially tubular and may have a predefined volume e.g. of about 20 ml. In an example, the receptacle 201 may be made of glass to prevent the degradation of the contained liquid fragrance.

The liquid fragrance container 200 comprises a pump mechanism 202 for dispensing a predetermined amount of liquid, e.g. 0.1 ml, when actuated. The pump mechanism 202 may be arranged on top of the fragrance container 200 and may be any of standards and/or commercially available mechanisms. Using a standard pump mechanism may be a low cost solution which may additionally provide a good accuracy, may reduce quality problems and may also prevent vacuum problems e.g. leakages. The pump mechanism 202 may be a manually actuated pump i.e. it may be actuated without a power source.

The fragrance container 200 may additionally comprise an actuating element such as a push button 204 to actuate the pump mechanism.

In an example, the button 204 may comprise a concave shape recess so as to adapt to the end of an external actuator, e.g. a plunger, of an external device (later on disclosed) to facilitate actuating the pump mechanism e.g. by providing a substantially matching shape to facilitate an efficient actuation of the pump mechanism.

The fragrance container 200 comprises an outlet conduit 203 coupled to the pump mechanism 202 to output the predefined amount of liquid when actuated. The conduit may be substantially tubular and may be made of plastic.

In an example, the outlet conduit 203 may comprise a diameter reducing element (not shown) that may reduce the diameter of the conduit so as to prevent or substantially avoid leakage problems. Such diameter reducing element may also reduce the entry of air into the conduit and enables the drawn liquid amount to be released with certain pressure when the pump mechanism is actuated. In an example, the diameter reducing element may be made of plastic.

Figure 3 shows in perspective a housing 100 for containing a liquid fragrance container e.g. according to the example of Figure 2. The housing 100 comprises a main body 101, a closure 102 and a bottom wall 103.

The main body 101 may be dimensioned to enable a fragrance container, such as the fragrance container 200 of Figure 2, to be inserted therein. In an example, the length of the main body may be e.g. of about 110 mm. The main body 101 is substantially tubular but any other suitable shape similar to the fragrance container to be housed may also be possible.

The housing 100 further comprises an adjusting element 110 (see Figure 1) to be inserted around the fragrance container. The adjusting element 110 may comprise an outer shape substantially corresponding to the shape of the main body 101, and an inner shape for matching with the shape of the fragrance container 200.

The cartridge further comprises an opening 104 aligned with the pump mechanism of the fragrance container, for allowing the pump mechanism to be actuated, and an output opening 105 in correspondence with the outlet conduit of the fragrance container.

The closure 102 may be fixedly mounted on the main body 101 e.g. by adhesive, and may comprise flanges 106 to facilitate the coupling (see Figure 1). The closure 102 comprises the opening 104 which may be sized to permit an external actuator, e.g. a plunger, of a dispensing device to pass through it and actuate the pump mechanism. Besides, the opening 104 may also be sized to restrict the access to the inside of the housing e.g. to avoid undesired manual actuations in which the released liquid amount may not be registered. In an example the opening 104 may be e.g. about 3 to 50 mm of diameter.

The bottom wall 103 comprises the outlet opening 105 which may be arranged substantially in the periphery of the bottom wall to facilitate the insertion of the outlet conduit 203 of a fragrance container 200 once the fragrance container is enclosed in the housing. The outlet opening 105 may be sized to enable a tight fitting of the outlet conduit.

In order to hold in position the outlet conduit of the fragrance container, the housing may further comprise an insert element 130 (see figure 1). The insert element 130 may be arranged in the vicinity of the outlet opening 105 e.g. at, near or around the opening, and may also be fixedly attached to the housing e.g. by an adhesive.

In an example, the housing 100, the adjusting element 110 and the insert element 130 may be made of plastic.

The housing 100 may comprise a predefined cross-section for arranging the cartridge in a predetermined position when inserted in a recess or a seat of an external device e.g. a dispensing device. In an example, the cross-section of the housing may be substantially D-shaped and rounded.

The housing 100 may further comprise an identification element 140 attached thereto e.g. placed on the cover. The identification element 140 may electronically store information related to the cartridge e.g. the identification number, the type of fragrance contained, the remaining fragrance volume, a description of the fragrance, etc.

Some of the data stored in the identification element 140, e.g. the remaining fragrance volume, may need to be re-written or updated after each use, the identification element may therefore be a read/write identification element.

In an example, the identification element may be a Radio Frequency Identification (RFID) tag.

Additionally, the housing 100 may comprise an authentication element 150 to be checked by an external sensor e.g. an antenna of a dispensing device, which may prevent the cartridge from being actuated in case the origin is not verified. The authentication element 150 may be attached to the housing e.g. on the closure to be more easily readable by an antenna.

In some examples, the authentication element 150 may be a symbol made of a security ink e.g. any of commercially available product, or a watermark.

In other examples, the authentication element 150 may comprise magnetic particles. In such examples, a predetermined percentage of magnetic particles may be added to the housing e.g. in the closure. To authenticate a cartridge, the proportion of particles may be read/measured by an antenna of the dispensing device and be afterwards compared e.g. by a control system, with a stored reference value. In an example, the magnetic particles may be any of commercially available particles e.g. Gravitech^{™}, Stattech^{™}, etc.

In an example, the magnetic particles may have a conductivity e.g. of about 100 Ω.

Referring back to the assembled cartridge, Figure 1 depicts a simplified and very schematic longitudinal view of cartridge 1 comprising a housing 100 according to the example of Figure 3 having a fragrance container 200 according to the example of Figure 2 inserted therein.

The fragrance container 200 may be a fragrance container according to any of the disclosed embodiments which comprises a receptacle 201, a pump mechanism 202, an outlet conduit 203 and may comprise an actuating element such as a push button 204 arranged on top of the pump mechanism.

Figure 1 shows a housing 100 which comprises the main body 101, the closure 102 that comprises the opening 104 and the bottom wall 103 which comprises the outlet opening 105. The housing 100 also comprises the adjusting element 110 which may snuggly hold the fragrance container inside the housing e.g. avoiding or substantially reducing the undesired movements of the fragrance container which may lead to its rupture. The size of the adjusting element 110 may be substantially similar to the dimensions of the inner cavity formed by the main body 101 and the bottom wall 103 thereby the movements of the adjusting element 110 would be substantially restricted or almost avoided inside the housing. In addition, the adjusting element 110 may comprise inwardly protruding portions to correspondingly engage the inner shape of the fragrance container, a snuggly fitting of the fragrance container may therefore be obtained.

The adjusting element 110 may also facilitate the automatization of the cartridge assembling process e.g. as due to the outer shape the adjusting element may facilitate the insertion of the fragrance container into the housing. In addition, to further facilitate the automatization, the adjusting element may arrange the fragrance container 200 in a predetermined position to place e.g. the outlet conduit in correspondence with the outlet opening, and enable a quicker assembly.

Moreover, the adjusting element may enable the arrangement of the fragrance container 200 in a predetermined position within the housing e.g. in a position in which the button 204 of the pump mechanism 202 is aligned with the opening 104 of the closure 102.

In addition, the housing 100, according to previously disclosed examples, may comprise an insert element 130 arranged in the vicinity of the outlet opening 105 for guiding and/or holding the outlet conduit 203 at the outlet opening for dispensing the liquid fragrance and avoid or substantially reduce leakage. In an example, the insert element may 130 comprise two side walls and a top wall having a hole which may be narrow enough let the conduit to be inserted therein and also to tightly hold the outlet conduit.

The fragrance container 200 may be inserted into the cavity formed by the bottom wall and the main body of the housing 100. The outlet conduit 203 of the fragrance container may be held by the insert element 130 at or substantially near the outlet opening 105 of the bottom wall. The adjusting element 110 may then be inserted to snuggly fit the fragrance container and may cause the fragrance container to be arranged in a specific position e.g. in which the pump mechanism to be aligned with the opening on the closure. In an alternative example, the adjusting element 130 may be coupled to the fragrance container 200 before introducing the latter in the housing.

The housing 100 may then be closed by coupling and attaching, e.g. by adhesive, the closure 102 to the main body 101 thereby assembling the whole cartridge 1.

Once the cartridge 1 is assembled, the dimensions of the opening 104 together with the arrangement of the button 204 and/or the pump mechanism 202 with respect to the opening 104 may prevent the pump mechanism from being (manually) accessible and/or from being undesirably actuated. That is, the pump mechanism 202 of the fragrance container, once inserted into the housing, may not be actuated out of a dispensing device but by an external actuator e.g. a plunger, of a dispensing device. The cartridge 1 may therefore be protected from undesired actuations e.g. by the customers, and in addition, unregistered liquid releases may be prevented.

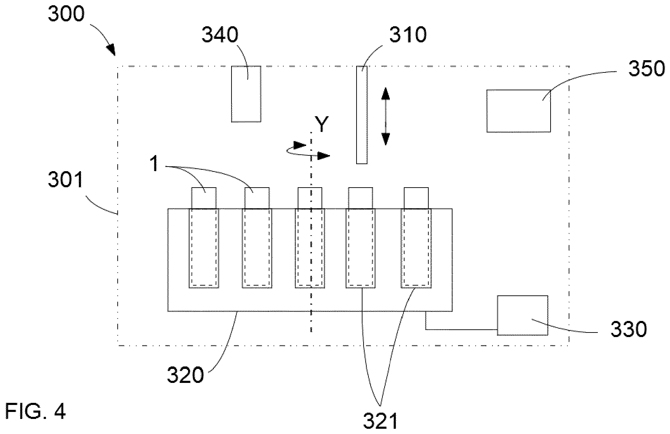

Figure 4 shows a very simplified and schematic longitudinal view of a fragrance dispensing device 300.

The dispensing device 300 may comprise a casing 301 in which a vertically movable actuator 310, e.g. a plunger, may be arranged. The actuator 310 may be an elongated rod sized to pass through the opening 104 of cartridges 1. When vertically displaced i.e. parallel to vertical axis Y, the vertically movable actuator 310 may therefore actuate the pump mechanism e.g. by pressing the bottom arranged on top of the fragrance container, and draw a predetermined amount of liquid. When more than the predetermined amount is to be released, the pump mechanism may be actuated as many times as necessary i.e. by subsequently displacing the vertically movable actuator.

The dispensing device 300 may also comprise a rotatable carrousel 320 that may be axially rotatable clockwise and/or anti-clockwise, (see the arrows) with respect to a vertical axis Y to increase the efficiency of the dispensing device as the carrousel may be rotated in the direction which requires less angular displacement in order to arrange a subsequent cartridge e.g. in the actuating position.

The carrousel 320 may comprise seats 321 for removably inserting a plurality of cartridges 1. The dispensing device 300 may comprise e.g. a motor 330 to provide rotatable movement to the carrousel 320 and move the cartridges to an actuating position in which the pump mechanism may be actuated.

Besides, the seats 321 may have a cross-section complementary to the cross-section of the cartridges 1 so as to enable arranging the cartridges in a predetermined manner e.g. an arrangement in which the pump mechanism of the fragrance container is to be aligned e.g. with a plunger of the device 300 when moved to the actuating position.

The dispensing device 300 may also comprise a read/write antenna 340 for writing and/or reading the identification elements, and also for reading the authentication elements arranged on the fragrance container housings 100.

In addition, the dispensing device 300 may further comprise a control system 350 in data communication with the read/write antenna, a data storage element, the actuators, the communication system, etc.

In use, the user may purchase or be provided with a plurality of cartridges comprising a plurality of fragrances, and also a dispensing device comprising a rotatably movable carrousel. Due to the cross-section of the seats, which may be complementary to the cross-section of the cartridges, each cartridge may be inserted into a seat of the carrousel in a predetermined position e.g. to be aligned with an actuator and/or an antenna of the dispensing device.

The user may select a fragrance composition or may create a fragrance to be dispensed via a user interface. The control system 350 of the dispensing device 300 may then check whether the fragrances required for the selected composition are available in the carrousel, and may inform the user if any element, i.e. a fragrance, is missing or if the amount of a fragrance is not enough for the selected composition.

To check the available fragrances and the remaining fragrance amount in each cartridge, the carrousel 320 may be axially rotated e.g. around a vertical axis Y. As the carrousel 320 is rotated, the read/write antenna, arranged in the dispensing device 300 may read an identification element that may store information related to each cartridge. The read/write antenna may be attached to the housing of each cartridge. The information stored in each identification element may comprise permanent data, i.e. data which may not need to be updated or re-written, such as the type of fragrance, a brief description of the fragrance, etc., and may also comprise non-permanent data which may be re-written in order to be updated e.g. the remaining fragrance amount. Besides, the control system 350 may associate each cartridge to a specific seat in the carrousel, and save the position of the cartridges e.g. in a data storage element.

Once the availability of required fragrances is verified, the control system 350 may then search for the cartridge comprising a first fragrance of the selected composition and rotate the carrousel, e.g. via a motor 330, so as to arrange such cartridge in a predetermined actuating position i.e. aligned with a vertically movable actuator, e.g. a plunger, of the dispensing device 300.

Before actuating the pump mechanism, the control system 350 may verify that such cartridge 1 is official or certified, i.e. confirm that the origin is valid. An antenna 340 of the dispensing device may read the authentication element of the cartridge e.g. a symbol made of a security ink or any other suitable material, and the control system may compare the read authentication element, e.g. a symbol, with a reference symbol in order to authenticate the cartridge.

Once the use of a cartridge has been allowed, the vertically movable actuator 310 may then be downwardly displaced and, as a consequence, the pump mechanism of the cartridge may be actuated e.g. by pressing a pushing button, thereby dispensing a predetermined amount of liquid fragrance.

In some examples, the selected composition may require an increased amount of a particular fragrance comparing to the predetermined amount dispensed by the pump mechanism when it is actuated. In such cases, the vertically movable actuator may be subsequently actuated as many times as necessary to dispense the required liquid quantity. For instance, if the required amount of a fragrance to be released for a selected composition is twice the predetermined amount drawn by the pump mechanism, the actuator may be actuated two subsequent times.

After actuating the pump mechanism of a cartridge, that is, after releasing the required amount of liquid, the control system 350 may register e.g. in a data storage element the number of times that the pump mechanism has been actuated in order to calculate and save, e.g. in a data storage element, the data of the remaining fragrance quantity in the cartridge. In addition, such information, i.e. the remaining liquid amount, may be re-written in the identification element thereby updating the information comprised in the cartridge. Each cartridge may therefore comprise an updated status which enables for example, retrieving the information offline i.e. without need to access a specific data storage element. Being able to update the information of the identification element also enables the possibility of using the cartridges in a different dispensing device as the varying information i.e. the information to be updated e.g. the remaining liquid amount, may be directly retrieved from each cartridge.

Once the required amount of liquid is released from the actuated cartridge, e.g. into a removable vessel (not shown), the control system may search the cartridge comprising the following fragrance of the selected composition and repeat the steps until the corresponding amount of all the fragrances of the composition is released.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by the scope of the claims that follow.

## Claims

1. A fragrance cartridge (1) for a fragrance dispensing device comprising:
a housing (100);
a liquid fragrance container (200) arranged inside the housing, the container comprising
a receptacle (201) for containing a fragrance liquid,
a pump mechanism (202) for drawing a predetermined amount of liquid fragrance from the receptacle after being actuated,
an outlet conduit (203) coupled to the pump mechanism to output the predetermined liquid amount from the container (200);
wherein the housing further comprises an output opening (105) in correspondence with the outlet conduit (203) of the fragrance container, and an opening (104) aligned with the pump mechanism (202) of the fragrance container, for allowing the pump mechanism to be actuated,
wherein the cartridge (1) comprises an internal adjusting element (110) arranged inside the housing (100) and having an inner shape substantially matching the outer shape of the fragrance container (200);
**characterized in that** the housing (100) further comprises
a substantially tubular main body (101) for containing the fragrance container (200),
a bottom wall (103) comprising the output opening (105) for the outlet conduit (203) of the fragrance container (200),
a closure (102) comprising the opening (104) to be aligned with the pump mechanism (202) of the fragrance container,
wherein the openings (104, 105) are arranged at opposite ends of the housing (100).

2. The fragrance cartridge according to claim 1, further comprising an insert element (130) in the vicinity of the output opening (105) to hold in position the outlet conduit (203) of the fragrance container with respect to the output opening.

3. The fragrance cartridge according to claim 2, further comprising a read/write identification element (140) attached to the housing (100).

4. The fragrance cartridge according to claim 3, wherein the identification element (140) is Radio Frequency Identification (RFID) tag.

5. The fragrance cartridge according to any of claims 1 - 4, further comprising an authentication element (150) arranged on the housing (100).

6. The fragrance cartridge according to claim 5, wherein the authentication element (150) comprises a security ink or magnetic particles.

7. The fragrance cartridge according to any of claims 1 - 6, wherein housing (100) is made of plastic.

8. A fragrance dispensing device comprising:
a rotatable carrousel (320) having a plurality of seats (321),
fragrance cartridges (1) according to any of claims 1 - 7 placed in the seats (321), the seats comprising an output orifice to be aligned with the output opening (105) of a cartridge, and wherein the seats have a shape complementary to the cross section of the cartridge;
a read/write antenna (340) for writing and/or reading identification elements (140) and authentication elements (150) arranged on the container housings (100), and
a vertically movable actuator (310) for actuating a cartridge (1).

## Patentansprüche

1. Eine Duftkartusche (1) für eine Duftabgabevorrichtung umfassend:
ein Gehäuse (100);
einen Flüssigduftbehälter (200), der innerhalb des Gehäuses angeordnet ist, wobei der Behälter Folgendes umfasst
eine Aufnahme (201) zum Aufnehmen einer Duftflüssigkeit,
einen Pumpmechanismus (202) zum Ansaugen einer vorbestimmten Menge flüssigen Duftstoffs aus der Aufnahme, nachdem er betätigt worden ist,
eine Auslassleitung (203), die mit dem Pumpmechanismus gekoppelt ist, um die vorbestimmte Flüssigkeitsmenge aus dem Behälter (200) auszugeben;
wobei das Gehäuse ferner eine Ausgangsöffnung (105) in Übereinstimmung mit der Auslassleitung (203) des Duftbehälters und eine Öffnung (104) umfasst, die mit dem Pumpmechanismus (202) des Duftbehälters ausgerichtet ist, um zu ermöglichen, dass der Pumpmechanismus betätigt wird,
wobei die Kartusche (1) ein inneres Einstellelement (110) umfasst, das innerhalb des Gehäuses (100) angeordnet ist und eine innere Form aufweist, die im Wesentlichen an die äußere Form des Duftbehälters (200) angepasst ist;
**dadurch gekennzeichnet, dass** das Gehäuse (100) weiterhin Folgendes umfasst
einen im Wesentlichen röhrenförmigen Hauptkörper (101) zum Aufnehmen des Duftbehälters (200),
eine Bodenwand (103), welche die Ausgangsöffnung (105) für die Auslassleitung (203) des Duftbehälters (200) umfasst,
einen Verschluss (102) umfassend die Öffnung (104), die mit dem Pumpmechanismus (202) des Duftbehälters auszurichten ist,
wobei die Öffnungen (104, 105) an gegenüberliegenden Enden des Gehäuses (100) angeordnet sind.

2. Die Duftkartusche nach Anspruch 1, ferner umfassend ein Einsatzelement (130) in der Nähe der Ausgangsöffnung (105), um die Auslassleitung (203) des Duftbehälters in Bezug auf die Ausgangsöffnung in Position zu halten.

3. Die Duftkartusche nach Anspruch 2, ferner umfassend ein an dem Gehäuse (100) befestigtes Lese-/Schreib-Identifikationselement (140).

4. Die Duftkartusche nach Anspruch 3, wobei das Identifikationselement (140) ein Radiofrequenz-Identifikations(RFID)-Tag ist.

5. Die Duftkartusche nach einem der Ansprüche 1 bis 4, ferner umfassend ein an dem Gehäuse (100) angeordnetes Authentifizierungselement (150).

6. Die Duftkartusche nach Anspruch 5, wobei das Authentifizierungselement (150) eine Sicherheitstinte oder magnetische Partikel umfasst.

7. Die Duftkartusche nach einem der Ansprüche 1 bis 6, wobei das Gehäuse (100) aus Kunststoff hergestellt ist.

8. Eine Duftabgabevorrichtung umfassend:
ein drehbares Karussell (320) mit einer Vielzahl von Sitzen (321),
Duftkartuschen (1) nach einem der Ansprüche 1 bis 7, die in den Sitzen (321) angeordnet sind, wobei die Sitze eine Ausgabeöffnung umfassen, die mit der Ausgangsöffnung (105) einer Kartusche auszurichten ist, und wobei die Sitze eine zum Querschnitt der Kartusche komplementäre Form haben;
eine Schreib-/Leseantenne (340) zum Schreiben und/oder Lesen von an den Behältergehäusen (100) angeordneten Identifikationselementen (140) und Authentifizierungselementen (150), und
ein vertikal bewegbares Betätigungselement (310) zum Betätigen einer Kartusche (1).

## Revendications

1. Une cartouche de parfum (1) pour un dispositif de distribution de parfum comprenant:
un boîtier (100) ;
un conteneur de parfum liquide (200) agencé à l'intérieur du boîtier, le conteneur comprenant
un réceptacle (201) pour contenir un parfum liquide,
un mécanisme de pompe (202) pour aspirer une quantité prédéterminée de parfum liquide du réceptacle après avoir été actionné,
un conduit de sortie (203) couplé au mécanisme de pompe pour sortir la quantité de liquide prédéterminée du conteneur (200) ;
dans lequel le boîtier comprend en outre une ouverture de sortie (105) en correspondance avec le conduit de sortie (203) du conteneur de parfum, et une ouverture (104) alignée avec le mécanisme de pompe (202) du conteneur de parfum, pour permettre au mécanisme de pompe d'être actionné,
dans lequel la cartouche (1) comprend un élément de réglage interne (110) agencé à l'intérieur du boîtier (100) et ayant une forme intérieure correspondant sensiblement à la forme extérieure du conteneur de parfum (200) ;
**caractérisée en ce que** le boîtier (100) comprend en outre
un corps principal essentiellement tubulaire (101) pour contenir le conteneur de parfum (200) ;
une paroi de fond (103) comprenant l'ouverture de sortie (105) pour le conduit de sortie (203) du conteneur de parfum (200),
une fermeture (102) comprenant l'ouverture (104) à aligner avec le mécanisme de pompe (202) du récipient de parfum,
dans lequel les ouvertures (104, 105) sont agencées à des extrémités opposées du boîtier (100).

2. La cartouche de parfum selon la revendication 1, comprenant en outre un élément d'insert (130) au voisinage de l'ouverture de sortie (105) pour maintenir en position le conduit de sortie (203) du conteneur de parfum par rapport à l'ouverture de sortie.

3. La cartouche de parfum selon la revendication 2, comprenant en outre un élément d'identification de lecture/écriture (140) attaché au boîtier (100).

4. La cartouche de parfum selon la revendication 3, dans laquelle l'élément d'identification (140) est une étiquette d'identification par radiofréquence (RFID).

5. La cartouche de parfum selon l'une quelconque des revendications 1 à 4, comprenant en outre un élément d'authentification (150) agencé sur le boîtier (100).

6. La cartouche de parfum selon la revendication 5, dans laquelle l'élément d'authentification (150) comprend une encre de sécurité ou des particules magnétiques.

7. La cartouche de parfum selon l'une quelconque des revendications 1 à 6, dans laquelle le boîtier (100) est réalisé en plastique.

8. Un dispositif de distribution de parfum comprenant :
un carrousel rotatif (320) ayant une pluralité de sièges (321)
des cartouches de parfum (1) selon l'une quelconque des revendications 1 à 7 situées dans les sièges (321), les sièges comprenant un orifice de sortie devant être aligné avec l'ouverture de sortie (105) d'une cartouche, et dans lequel les sièges ont une forme complémentaire à la section transversale de la cartouche ;
une antenne de lecture/écriture (340) pour écrire et/ou lire des éléments d'identification (140) et des éléments d'authentification (150) agencés sur les boîtiers de conteneur (100), et
un actionneur mobile verticalement (310) pour actionner une cartouche (1).
